Europäisches Patentamt

European Patent Office

Office européen des brevets

(11) Publication number: **0 407 188 A1**

# EUROPEAN PATENT APPLICATION

(21) Application number: **90307344.3**

(22) Date of filing: **05.07.90**

(51) Int. Cl.⁵: **G01N 33/58, G01N 33/533**

(30) Priority: **07.07.89 GB 8915654**

(43) Date of publication of application:
**09.01.91 Bulletin 91/02**

(84) Designated Contracting States:
**AT BE CH DE DK ES FR GB GR IT LI NL SE**

(71) Applicant: **UNILEVER PLC**
**Unilever House Blackfriars P.O. Box 68**
**London EC4P 4BQ(GB)**

(84) **GB**

Applicant: **UNILEVER NV**
**Burgemeester s'Jacobplein 1 P.O. Box 760**
**NL-3000 DK Rotterdam(NL)**

(84) **BE**

(72) Inventor: **Bosley, John Anthony**
**43 Kettering Road, Islip**
**Kettering, Northamptonshire NN14 3JT(GB)**
Inventor: **Davidson, Ian William**
**Braeside, Springhill**
**Little Staughton, Bedfordshire MK44**
**2BS(GB)**
Inventor: **Mundill, Paul Henry Charles**
**4 Brookside, Stanwick**
**Wellingborough, Northamptonshire NN9**
**6PW(GB)**
Inventor: **Richards, Ian**
**6 Lansdown Place West**
**Bath BA1 5EZ(GB)**

(74) Representative: **Green, Mark Charles et al**
**Unilever PLC Patent Division P.O. Box 68**
**Unilever House Blackfriars**
**London EC4P 4BQ(GB)**

(54) **Detectable particles.**

(57) A reagent useful in immunoassays and the like comprises micro particles of latex (polystyrene) containing therein a water-insoluble fluorophore such as a laser dye, with the fluorophore being essentially absent from the particle surfaces. The particles can be sensitised with specific binding reagents such as antibodies. The fluorophore-containing particles exhibit constant fluorescent yield, and provide improved labels useful in sensitive quantitative assays.

EP 0 407 188 A1

## DETECTABLE PARTICLES

The present invention relates detectable water-insoluble particles useful, for example, as labels which can provide a detectable signal to indicate the result of an analysis such as a specific binding assay, especially an immunoassay. The invention particularly relates to such particles which are fluorescent.

Fluorescent materials have been used previously as assay labels. Conventional fluorophores are excited by ultraviolet light and emit visible light. Unfortunately, natural materials such as body fluids to which assays need to be applied contain many components which also absorb UV light and act as fluorophores, and such "autofluorescence" can generate an inconvenient background signal and reduce the sensitivity of an assay. To avoid this problem, the use of so-called "laser dyes" as fluorescent labels has been proposed, for example by Soini and Hemmila in Clin. Chem. 25/3 (1979) pages 353-361. According to Soini and Hemmila the general requirements for a fluorescence probe are an emission wavelength between 500 and 700 nm and a Stokes' shift of greater than 50 nm, preferably more than 100 nm. In the immunoassay systems that they describe, the probe should also be highly soluble in water.

In our UK patent application GB 2204398A we describe immunochromatographic devices which use particulate labels. Concentration of a particulate label in a detection zone on a porous carrier material such as nitrocellulose generates a detectable assay result. The particulate labels used in GB 2204398A are preferably "direct labels" i.e. coloured particles such as coloured latex microspheres or dye sol particles which are detectable by eye when present in sufficient concentration. The use of such coloured direct labels in immunochromatographic assays enables the user to observe a qualitative or semi-quantitative result easily. However, direct labels are unsuitable if a sensitive quantitative assay result is required.

In one embodiment, the invention provides a water-insoluble solid particle containing entrapped therein a fluorophore, the fluorophore being essentially absent from the particle surface.

A more preferred embodiment is a latex particle containing entrapped therein a water-insoluble fluorophore, the fluorophore being essentially absent from the particle surface.

A further embodiment is a reagent comprising one or more particles as defined in either of the immediately preceding two paragraphs, in an aqueous medium such as distilled water or an aqueous buffer solution, the aqueous medium also being essentially free from fluorophore (whether in solution or as a sol).

Another embodiment comprises such particles whose surfaces have been sensitised with material capable of participating in a specific binding reaction, such as antigenic material, haptens, antibodies and lectins. Such sensitised particles can be in aqueous media, or in dried form, e.g. as a dried, potentially-mobile labelled reagent on a testing apparatus such as a dry test strip comprising a bibulous carrier material.

The optical characteristics of the fluorophore can be affected by its chemical environment; by shielding the fluorophore within the particle such unwanted influences can be minimised and a more consistent and reliable emission signal can be observed. By ensuring that the fluorophore is entrapped within the particle but essentially absent from the surface of the particle, the risk of external influence on the optical characteristics is reduced further, and moreover, there is less risk that fluorophore may leach out of the particle into its surroundings with resulting loss of fluorescent sensitivity. If fluorophore is present on the particle surface, it may block useful binding sites on the surface, and if it leaches off during use of the particle during an immunoassay, for example, it may expose additional binding sites and introduce undesirable variability in assay performance.

The invention provides a process for the preparation of fluorophore-containing particles, wherein pre-formed particles as an aqueous dispersion are contacted with a solution of a water-insoluble fluorophore in a non-aqueous solvent capable of swelling the particle material, for a time sufficient to allow the particles to absorb the fluorophore, the non-aqueous solvent is removed from the aqueous dispersion, and the particles are washed (repeatedly if necessary) in a liquid capable of solubilising the fluorophore without penetrating the particles.

In particular, the invention provides a process for the preparation of fluorophore-containing latex particles, wherein pre-formed latex particles as an aqueous dispersion are contacted with a solution of water-insoluble fluorophore in a water-immiscible solvent capable of swelling the latex particles, for a time sufficient to allow the particles to absorb the fluorophore, the solvent is removed from the aqueous dispersion, and the particles are washed (repeatedly if necessary) in a liquid capable of solubilising the fluorophore without penetrating the latex.

Preferably, the removal of superficial fluorophore by particle washing should be continued until the level of fluorophore drops below its detectable limit, ie. essentially no further fluorophore can be observed in the wash liquor.

The water-inmiscible solvent is preferably chloroform, but dichloromethane, xylene, toluene and benzene can be used.

The washing liquid is preferably an alcohol/water mixture. The proportions of alcohol to water may need to be chosen having regard to the relative solubility of the fluorophore. Pure alcohol, such as ethanol, will penetrate latex, so should not be used. A 1:1 mix of ethanol and water is usually ideal.

After being washed free from superficial fluorophore, the particles are conveniently stored in the form of an aqueous dispersion containing no organic solvent.

After adequate washing, the particles will exhibit a constant fluorescent yield. This can be determined using any standard spectrofluorometer, and an example of a suitable procedure is given below. Constant fluorescent yield is very important for batch-to-batch consistency in immunoassays.

By the process of the invention it is possible to prepare particles containing any level of fluorophore up to the saturation level, and it is possible to produce a range of particle reagents exhibiting different fluorescent yields.

Preferably the fluorophore is a laser dye. Preferred fluorophores 3-(2'-benzothiazolyl)-7-diethylamino coumarin (usually referred to as Coumarin 6), 1,2,4,5,3H, 8H, 10H-tetrahydro-8-trifluoromethyl[1]-benzopyrano-(9,9a,1-gh) quinolizin-10-one (usually referred to as Coumarin 153), and 4-(dicyanomethylene)-2-methyl-6-(p-dimethylaminostyryl)-4H-pyran (usually referred to as DCM).

Latex (polymer) particles for use in immunoassays are available commercially. These can be based on a range of synthetic polymers, such as polystyrene, polyvinyltoluene, polystyrene-acrylic acid, polyacrolein, and poly(meth)acrylate esters and their copolymers. The monomers used are normally water-insoluble, and are emulsified in aqueous surfactant so that monomer droplets and/or micelles are formed, which are then induced to polymerise by the addition of initiator to the emulsion. Substantially spherical monodisperse polymer particles are produced. By controlling the conditions, a variety of size ranges can be provided. For the purposes of the present invention, such latex particles preferably have a maximum dimension of less than about 0.5 micron.

The fluorophore-containing particles of the invention can be used in un-sensitised form, for example as size standards in microscopy. However, the widest envisaged use of the particles will be as labels in specific binding assays, where the particles will be sensitised with reagents such as antigens, antibodies, or other specific binding reagents such as enzymes and their co-factors, avidin, and biotin.

By way of example only, the preparation and properties of typical fluorescent latex particles in accordance with the invention are described below.

## Preparation of monodisperse latex particles containing fluorophore

### a) Preparation of latex

Solution A - continuous phase :
270 ml distilled water
0.114 g sodium tetraborate decahydrate
0.450 g sodium dodecyl sulphate
Solution B - monomer phase :
90 g styrene monomer
1.5 g low mol. wt. polystyrene

Solution A is deoxygenated by sparging with argon gas for 10 minutes. Solution B is then poured into solution A and homogenised using an Ultra-Turrax homogeniser at 24,000 rpm for 60 seconds. The resultant emulsion is placed in a glass reaction flask equipped with a condenser and an overhead stirrer, and the temperature raised to 80°C in a water bath. The reaction mix is then sparged with argon for a further 5 minutes whilst stirring at approximately 200 rpm. An initiator solution comprising 0.5 g sodium persulphate in 10 mls distilled water is then added and the polymerisation allowed to proceed for 20 hours. An equivalent amount of initiator is then added and the polymerisation allowed to proceed for a further 4 hours. The latex suspension is allowed to cool and then filtered through a bed of glass wool. Any residual monomer is then removed on a rotary evaporator to yield a monodisperse latex suspension with a particle diameter of approximately 0.2 micron.

Sodium dodecyl sulphate surfactant, initiator residues and sodium tetraborate can be removed from the latex if required by a combination of slurrying with DOWEX Ion-Retardation resin followed by dialysis.

3

### b) Incorporation of fluorophore

The polymer solids content of the aqueous latex dispersion is accurately determined by evaporating a known volume to dryness.

A solution of Coumarin 153 in chloroform is prepared to give a concentration of approximately 40 mg/ml. The aqueous latex dispersion is placed in a polyethylene container, diluted with water as necessary to give a concentration of approximately 15mg polymer solids per ml dispersion, and a volume of the fluorophore solution added such that 1 microlitre is added for each 1 mg polymer solids present. The suspension is gently swirled on an orbital stirrer for 18 hours at room temperature, the chloroform is then removed on a rotary evaporator at approximately 60°C. If required, a further aliquot of fluorophore solution can be added and the cycle repeated to give the desired incorporation. The latex is then filtered to remove any agglomerated polymer particles and an accurate solids content determined by evaporation. An aliquot of suspension is then diluted to give a concentration of 0.1 mg/ml and the fluorescence measured on a spectrofluorimeter.

Excitation wavelength = 423nm

Emission wavelength = 483nm for Coumarin 153

### c) Removal of excess fluorophore

The process as described above will yield polymer particles containing entrapped fluorophore. However, the preparation will also contain an appreciable amount of fluorophore present as a sol in the aqueous phase and some fluorophore weakly bound to the surface of the particles.

To remove this, the latex prepared above is diluted with an equal volume of absolute ethanol and placed in an Amicon diafiltration apparatus equipped with an Amicon YM100 membrane and diafiltered against an ethanol:water (1:1) mixture until the filtrate is colourless.

If any polymer particles have packed down onto the membrane they can be resuspended by gentle sonication. The ethanol is then removed under reduced pressure at 80 C. The preparation is then placed in a sonic water bath for 5 minutes to break-up any weak agglomerates and filtered through a Whatman 541 filter paper. The fluorescent latex preparation is stored in polypropylene bottles in the dark at 4°C. If required sodium azide can be added at 0.01% w/v to inhibit microbial growth.

### d) Determination of polymer solids content

A 1ml aliquot of fluorescent latex is pipetted into a pre-weighed glass vial. This is then heated in a forced air oven for 16 hours at 90°C and then for 2 hours at 110 C. The vial is allowed to cool to room temperature over phophorous pentoxide and then reweighed. This test is carried out in duplicate and the solids content is expressed in g/ml.

### e) Determination of fluorescence yield

A suspension of fluorescent latex is diluted with distilled water to give a solids content of 0.1 mg/ml. The fluorescence is then measured on a Baird Nova Spectrofluorimeter set on range 5 and with an excitation wavelength of 423 nm and an emission wavelength of 483 nm. Using such equipment, the value obtained will normally be in the range 150-170 fluorescence units. It should however be noted that these units are not absolute and will depend on the spectrofluorimeter being used, consequently they should only be used on a comparative basis.

### f) Determination of fluorophore content

Using the solids content obtained a solution of latex in tetrahydrofuran is prepared to give a concentration of 0.1 mg/ml. The absorbance of 410 nm is measured on a spectrophotometer and related to a standard curve for Coumarin 153 in tetrahydrofuran. The dye content of the latex is expressed as mgs. Coumarin 153 per g. polymer solids.

Particles can be prepared with Coumarin 153 contents up to 80 mgs/g by this technique, although

4

values of 30 mgs/g are normally adequate for most uses.

The Coumarin content of these particles is normally approximately 30 mgs per g. polymer solids. This represents an incorporation of approximately 95% (2 passes of 16mgs Coumarin = 32 mgs/g added). We have measured a range of degrees of incorporation depending on the number of fluorophore passes and the concentration of the fluorophore solution used, this range is 70-95%.

Fluorophore contents achieved are apparently independent of particle size over a range of 200-500nm particle diameter. This suggests that the fluorophore is evenly distributed throughout the particle, with the exception of the washed surface.

## g) Reproducibility of Coumarin Incorporation

A polystyrene latex dispersion prepared as in paragraph (a) above was shaken with a Coumarin 153 solution in chloroform (0.25 ml @ 40 mg/ml.) for 16 hours. The chloroform was removed under reduced pressure at $60°C$ and the sample diluted with an equal volume of absolute ethanol. This was diafiltered against ethanol:water (1:1) until the dialysate was colourless. The ethanol was removed by diafiltration against distilled water.

This was carried out on 5 separate samples using the same stock solution of fluorophore.

| Results | | |
|---|---|---|
| Sample | Fluorescence (1) | Absorbance 410nm (2) |
| 1 | 128 | 0.162 |
| 2 | 124 | 0.164 |
| 3 | 132 | 0.157 |
| 4 | 132 | 0.145 |
| 5 | 126 | 0.161 |
| Average | 128.4 (2.8%) | 0.158 (4.8%) |
| Notes: | | |

1. Spectrofluorimeter range 5, 0.1 mg.ml suspension. The fluorescence yields should be used on a comparative basis only, not as absolute values.
2. THF solution of latex at 0.1 mg.ml.

An absorbance value of 0.158 = 30 mgs Coumarin per g. latex solids. This represents a dye incorporation of 75%.

## Particle sensitisation

The fluorescent latex particles may be sensitised with protein, and in particular antibody, to provide reagents for use in immunoassays. For example, polystyrene beads of about 0.3 micron diameter may be sensitised with anti-alpha human chorionic gonadotrophin, in the process described below:

0.5ml (12.5mg solids) of suspension is diluted with 1 ml of 0.1M borate ph 8.5 in an Eppendorf vial. These particles are washed four times in borate buffer, each wash consisting of centrifugation for 3 minutes at 13000 rpm in an MSE microcentrifuge at room temperature. The final pellet is resuspended in 1ml borate buffer, mixed with $300\mu g$ of anti-alpha hCG antibody, and the suspension is rotated end-over-end for 16-20 hours at room temperature. The antibody-latex suspension is centrifuged for 5 minutes at 13000 rpm, the supernatant is discarded and the pellet resuspended in 1.5mls borate buffer containing 0.5 milligrammes bovine serum albumin. Following rotation end-over-end for 30 minutes at room temperature, the suspension is washed three times in 5mg/ml BSA in phosphate buffered saline pH7.2, by centrifugation at 13000 rpm for 5 minutes. The pellet is resuspended in 5mg/ml BSA/5% (w/v) glycerol in phosphate buffered saline pH7.2 and stored at $4°$ until used.

**Claims**

1. A Water-insoluble solid particle containing entrapped therein a fluorophore, the fluorophore being essentially absent from the particle surface.

2. A latex particle containing entrapped therein a water-insoluble fluorophore, the fluorophore being essentially absent from the particle surface.

3. A reagent comprising a plurality of particles according to claim 1 or claim 2, in an aqueous medium which is also essentially free from fluorophore.

4. A particle or reagent according to any one of the preceding claims, wherein the particle or particles are sensitised with material capable of participating in a specific binding reaction.

5. A particle or reagent according to any one of the preceding claims, wherein the fluorophore is a laser dye.

10. A process for the preparation of fluorophore-containing particles, wherein pre-formed particles as an aqueous dispersion are contacted with a solution of a water-insoluble fluorophore in a non-aqueous solvent capable of swelling the particle material, for a time sufficient to allow the particles to absorb the fluorophore, the non-aqueous solvent is removed, and the particles are washed (repeatedly if necessary) in a liquid capable of solubilising the fluorophore without penetrating the particles.

11. A process for the preparation of fluorophore-containing latex particles, wherein pre-formed latex particles as an aqueous dispersion are contacted with a solution of water-insoluble fluorophore in a water emmissible solvent capable of swelling the latex particles, for a time sufficient to allow the particles to absorb the fluorophore, the solvent is removed, and the particles are washed (repeatedly if necessary) in a liquid capable of solubilising the fluorophore without penetrating the latex.

6. A reagent comprising a plurality of latex particles containing entrapped therein a fluorophore, which particles exhibit a constant fluorescent yield.

7. A reagent for use in immunoassays, comprising an aqueous dispersion of sensitised latex particles having a maximum dimension of less than about 0.5 micron, the particles containing entrapped therein a laser dye and exhibiting a constant fluorescent yield.

8. Use of a particle or reagent according to any one of the preceding claims, in an immunoassay.

9. A dried, potentially-mobile labelled reagent in a testing apparatus such as a dry test strip comprising a bibulous carrier material, wherein the label is a fluorophore-containing particle as claimed in any one of claims 1, 2, 4 or 5.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-308233 (EASTMAN KODAK COMPANY)<br>* column 2, lines 31 - 40 *<br>* column 4, lines 50 - 61 *<br>* column 11, lines 25 - 33 *<br>* column 13, line 10 - column 15, line 10 * | 1, 2, 4, 5 | G01N33/58<br>G01N33/533 |
| X<br>D | EP-A-291194 (UNILEVER NV)<br>* page 12, line 35 - page 13, line 5 *<br>& GB-A-2204398 | 1-4, 6-11 | |
| X | EP-A-280556 (EASTMAN KODAK COMPANY)<br>* page 1, line 44 - page 2, line 7 *<br>* page 4, lines 41 - 64 *<br>* page 9, lines 8 - 18 * | 1-4, 7-9 | |
| D,A | CLINICAL CHEMISTRY.<br>vol. 25, no. 3, 1979, WINSTON US<br>pages 353 - 361; E.Soini et al.:<br>"Fluoroimmunoassay: Present status and key problems"<br>* the whole document * | 1, 5 | |
| A | EP-A-2963 (EASTMAN KODAK COMPANY)<br>* the whole document * | 1-4, 8-11 | **TECHNICAL FIELDS SEARCHED (Int. Cl.5 )**<br><br>G01N |
| A | EP-A-212989 (EASTMAN KODAK COMPANY)<br>* page 3, lines 1 - 13 *<br>* page 5, lines 20 - 24 * | 1, 8, 9 | |
| A | EP-A-201211 (WHITTAKER CORPORATION)<br>* the whole document * | 1-10 | |

The present search report has been drawn up for all claims

| Place of search | Date of completion of the search | Examiner |
|---|---|---|
| BERLIN | 05 OCTOBER 1990 | DE KOK A.J. |